(19) Europäisches Patentamt
European Patent Office
Office européen des brevets

(11) Veröffentlichungsnummer : **0 347 380 B1**

(12) # EUROPÄISCHE PATENTSCHRIFT

(45) Veröffentlichungstag der Patentschrift :
**10.02.93 Patentblatt 93/06**

(51) Int. Cl.$^5$ : **C07C 209/74**

(21) Anmeldenummer : **89810428.6**

(22) Anmeldetag : **07.06.89**

(54) **Verfahren zur Herstellung von 4-Bromanilin-hydrobromiden.**

(30) Priorität : **14.06.88 US 206551**

(43) Veröffentlichungstag der Anmeldung :
**20.12.89 Patentblatt 89/51**

(45) Bekanntmachung des Hinweises auf die Patenterteilung :
**10.02.93 Patentblatt 93/06**

(84) Benannte Vertragsstaaten :
**AT BE CH DE ES FR GB GR IT LI LU NL SE**

(56) Entgegenhaltungen :
**EP-A- 0 052 817**
**FR-A- 2 288 083**
**FR-A- 2 424 906**
**GB-A- 2 098 210**

(56) Entgegenhaltungen :
**US-A- 3 962 336**
**HOURBEN-WEYL "METHODEN DER ORGANISCHEN CHEMIE", Band V/4, 4. Auflage, "Halogenverbindungen", 1960, Georg Thieme VerlagStuttgart, DE**
**BERICHTE DER DEUTSCHEN CHEMISCHEN GESELLSCHAFT, 33, Jahrgang, Band II, 1900, Seiten 1967-1975; E. FISCHER et al: "Über dieBildung quaternärer Ammoniumverbindungen bei den gebromten Homologen des Anilins"**

(73) Patentinhaber : **CIBA-GEIGY AG**
**Klybeckstrasse 141**
**CH-4002 Basel (CH)**

(72) Erfinder : **Hässig, Robert, Dr.**
**Gänsackerring 3**
**CH-5264 Gipf-Oberfrick (CH)**

EP 0 347 380 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von 4-Bromanilin-hydrobromiden der Formel I

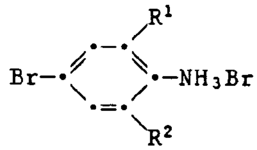

(I)

in welcher die Reste $R^1$ und $R^2$ unabhängig voneinander $C_1$-$C_{10}$-Alkyl oder $C_3$-$C_7$-Cycloalkyl bedeuten.

Die Verbindungen der Formel I sowie die zugrundeliegenden Amine sind wichtige Zwischenprodukte bei der Herstellung von insektizid wirksamen Thioharnstoff- und Carbodiimidderivaten, wie sie beispielsweise in der US-Patentschrift Nr. 4 328 247 und in der Europäischen Patentanmeldung Nr. 0 175 649 beschrieben sind.

In den Definitionen ist unter Alkyl geradkettiges oder verzweigtes Alkyl zu verstehen; z.B.: Methyl, Ethyl, n-Propyl, i-Propyl oder die vier isomeren Butyl, n-Pentyl oder die isomeren Pentyl, Hexyl oder die isomeren Hexyl, sowie Heptyl, Octyl, Nonyl und Decyl einschliesslich deren Isomere. Bevorzugt sind Alkylreste mit 1 bis 4 Kohlenstoffatomen, besonders bevorzugt sind dabei Methyl, Ethyl und i-Propyl.

Bei den als Substituenten $R^1$ und $R^2$ in Betracht kommenden Cycloalkylen handelt es sich beispielsweise um Cyclopropyl, Cyclobutyl, Cyclopentyl, Cyclohexyl oder Cycloheptyl. Diese können auch ein- oder mehrfach mit einem Alkylrest substituiert sein. Als bevorzugte Substituenten seien Cyclopentyl und Cyclohexyl genannt, ganz besonders bevorzugt ist Cyclopentyl.

Es ist bekannt, dass bei der Einwirkung von Brom auf 2,6-Dialkylaniline überwiegend 4-Brom-2,6-dialkylanilin entsteht. Wegen der grossen Reaktivität dieser Verbindungen verläuft die Reaktion nicht selektiv, was die Bildung von je nach Reaktionsbedingungen verschiedenen Nebenprodukten zur Folge hat. So wird zum Beispiel bei der Einwirkung von Brom auf 2,6-Dimethylanilin in Gegenwart von Eisessig 4-Brom-2,6-dimethylanilin in einer Ausbeute von 80 - 85 % der Theorie erhalten (Chem. Ber. (1901), 34, 2242). Bei der Bromierung von 2,6-Dimethylanilin in stark saurem Medium werden neben 4-Brom-2,6-dimethylanilin stets erhebliche Mengen 3-Brom-2,6-dimethylanilin gebildet (Chem. Ber. (1900), 33, 1967). Weiterhin ist aus der publizierten britischen Patentanmeldung GB A 2 098 210 bekannt, dass bei der Halogenierung von N-Alkyl-2,6-dialkylanilinen in Gegenwart von Lewis-Säuren, beispielsweise $AlCl_3$, das Halogen sogar selektiv in 3-Stellung eintritt.

In der US-A-3 962 336 wird die Chlorierung von 2-Toluidinhydrochlorid in einem Halogenkohlenwasserstoff zu 5-Chlor-2-toluidin in mässigen Ausbeuten beschrieben. In der FR-A-2 288 083 wird ein Verfahren zur Chlorierung von kernsubstituierten Anilinen vorgeschlagen, das zu Gemischen von Stellungsisomeren oder Anilinen mit unterschiedlichem Halogenierungsgrad führt. In der EP-A-0 052 817 ist ein Verfahren zur Chlorierung von 2,6-Dialkylanilinen zu 4-Chlor-2,6-dialkylanilinen in einem inerten organischen Lösungsmittel beschrieben, wobei kein vollständiger Umsatz und daher nur mässige Ausbeuten erzielt werden.

Abgesehen von der unbefriedigenden Ausbeute besteht ein wesentlicher Nachteil der bekannten Verfahren darin, dass die gewünschten 4-Brom-2 ,6-dialkylaniline in ungenügender Reinheit gebildet werden, sodass eine zusätzliche Reinigung der Produkte durch Destillation erforderlich wird. Diese ist jedoch im Hinblick auf die thermische Labilität der Brom-Aniline problematisch. Die Durchführung der Bromierung in Eisessig ist mit dem zusätzlichen Nachteil verbunden, dass bei der Aufarbeitung des Reaktionsgemisches eine verdünnte, wässrige Lösung von Natriumacetat entsteht, aus der die als Lösungsmittel verwendete Essigsäure nur mit sehr grossem Aufwand zurückgewonnen werden kann.

Mit den bekannten Verfahren ist es bisher nicht möglich, 4-Brom-2,6-dialkylaniline in befriedigender Weise herzustellen. Es ist daher das Ziel der vorliegenden Erfindung, ein Verfahren bereitzustellen, das die Herstellung von 4-Brom-2,6-dialkylanilinen in einfacher Weise sowie in guter Ausbeute und Reinheit ermöglicht.

Es wurde überraschenderweise gefunden, dass 4-Brom-2,6-dialkylanilinhydrobromide der Formel I durch Einwirkung von Brom auf 2,6-Dialkylanilin-hydrohalogenide der Formel II in einem inerten Lösungsmittel in nahezu quantitativer Ausbeute gebildet werden. Die erfindungsgemäss hergestellten 4-Brom-dialkylanilin-hydrobromide der Formel I können auf einfache Weise in die entsprechenden 4-Brom-2,6-dialkylaniline überführt werden.

Gemäss vorliegender Erfindung wird daher vorgeschlagen, 4-Brom-2,6-dialkylanilin-hydrobromide der Formel I in der Weise herzustellen, dass man ein 2,6-Dialkylanilin-hydrohalogenid der Formel II

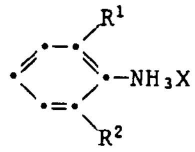

(II)

in welcher $R^1$ und $R^2$ die unter Formel I angegebene Bedeutung haben und X für Chlor oder Brom steht, in Gegenwart eines inerten, organischen Lösungsmittels mit Brom zu einer Verbindung der Formel I umsetzt.

Ausgangsverbindungen und Endprodukte des erfindungsgemässen Verfahrens sind bekannt. Die Anilinhydrohalogenide der Formel II können aus den entsprechend substituierten Anilinen durch Hydrohalogenierung gewonnen werden. Diese Aniline sind bekannt und nach bekannten Methoden herstellbar. Die Hydrohalogenierung von Anilinen lässt sich beispielsweise vorteilhaft mit Chlor- oder Bromwasserstoff in Gegenwart von Cyclohexan bei Raumtemperatur durchführen.

Gewünschtenfalls können die nach dem erfindungsgemässen Verfahren hergestellten 4-Bromanilin-hydrobromide der Formel I nach in der Technik üblichen Verfahren durch Einwirkung wässriger Basen in die entsprechenden 4-Bromaniline überführt werden.

Das erfindungsgemässe Verfahren wird vorteilhaft unter Atmosphärendruck bei Temperaturen von -20°C bis +150°C, wobei Temperaturen von -5°C bis +100°C bevorzugt werden, in einem inerten, mit Wasser nicht mischbaren Lösungsmittel durchgeführt. Als Lösungsmittel kommen offenkettige oder cyclische gesättigte Kohlenwasserstoffe sowie gesättigte Halogenkohlenwasserstoffe in Betracht. Geeignete Lösungsmittel sind beispielsweise Hexan, Cyclohexan, Methylcyclohexan, Dichlormethan, Trichlormethan, Tetrachlormethan oder 1,2-Dichlorethan. Bevorzugte Lösungsmittel sind Cyclohexan und 1,2-Dichlorethan.

Insbesondere eignet sich das erfindungsgemässe Verfahren zur Herstellung von 4-Brom-2,6-dialkylanilin-hydrobromiden der Formel I, in welcher $R^1$ und $R^2$ jeweils für Methyl, Ethyl oder i-Propyl oder $R^1$ für i-Propyl und $R^2$ für Cyclopropyl steht.

Gemäss einer bevorzugten Variante des erfindungsgemässen Verfahrens werden die 4-Bromanilin-hydrobromide der Formel I in der Weise hergestellt, indem man ein Anilinhydrohalogenid der Formel II in Gegenwart von 1,2-Dichlorethan oder Cyclohexan bei einer Temperatur von -5° bis +100°C mit Brom umsetzt.

Durch das erfindungsgemässe Verfahren wird es möglich, 4-Brom-2,6-dialkylaniline in nahezu quantitativer Ausbeute herzustellen. Die gegenüber den bekannten Verfahren deutliche Steigerung der Ausbeute muss als überraschend angesehen werden, da gemäss Literaturangaben (vgl. Houben-Weyl, Methoden der org. Chemie V/4, 236, 274 ff. (1960)) die Selektivität der Bromierung in 4-Stellung bei Anilinsalzen geringer ist als bei den freien Basen. Als zusätzlicher Vorteil des erfindungsgemässen Verfahrens ist die Verwendung eines inerten, organischen, mit Wasser nicht mischbaren Lösungsmittels anzusehen, welches die Isolierung der freien Anilinbasen aus dem Reaktionsgemisch in wesentlich einfacherer Weise als bei den mit Essigsäure arbeitenden Prozessen ermöglicht.

Das erfindungsgemässe Verfahren wird durch die folgenden Beispiele näher erläutert.

Beispiel 1: Herstellung von 4-Brom-2,6-diisopropylanilin-hydrobromid

Zu einer Aufschlämmung von 24,1 g 2,6-Diisopropylanilin-hydrochlorid in 250 ml 1,2-Dichlorethan werden bei 0°C innerhalb von einer Stunde 19,1 g Brom zugetropft. Nach einstündigem Rühren bei 0°C wird die Reaktionsmischung filtriert, der Filterrückstand mit 1,2-Dichlorethan gewaschen und anschliessend bei 40°C im Vakuum getrocknet. Man erhält 36,8 g (97 % d. Th.) 4-Brom-2,6-diisopropylanilin-hydrobromid in Form gelber Kristalle. Nach Umkristallisation aus Essigester beträgt der Schmelzpunkt 235°C bis 237°C.

Beispiel 2:

Zu einer Aufschlämmung von 21,4 g 2,6-Diisopropylanilinhydrochlorid in 150 ml Cyclohexan werden bei 70°C innerhalb von zwei Stunden 19 g Brom tropfenweise zugesetzt. Der entstehende Chlorwasserstoff entweicht dabei kontinuierlich. Nach Abkühlen der Reaktionsmischung auf 10°C wird filtriert, der Filterrückstand mit Cyclohexan gewaschen und im Vakuum bei 40°C getrocknet. Man erhält 34,1 g 4-Brom-2,6-diisopropylanilin-hydrobromid (99,9 % d. Th) in Form gelber Kristalle mit einem Reinheitsgrad von 98,7 % und einem Schmelzpunkt von 234°C bis 235°C (Verbindung Nr. 1).

Entsprechend den vorstehend beschriebenen Arbeitsweisen werden auch die folgenden Verbindungen der Formel I erhalten:

| Verbindung Nr. | $R^1$ | $R^2$ | Schmelzpunkt |
|---|---|---|---|
| 2 | $CH_3$ | $CH_3$ | 241 - 242°C |
| 3 | $C_2H_5$ | $C_2H_5$ | 228 - 231°C |
| 4 | $C_3H_7(i)$ | 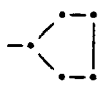 | 256 - 260°C |

## Patentansprüche

1. Verfahren zur Herstellung von 4-Bromanilin-hydrobromiden der Formel I

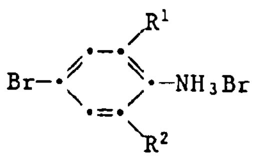

(I)

worin
$R^1$ und $R^2$ unabhängig voneinander $C_1$-$C_{10}$-Alkyl oder $C_3$-$C_7$-Cycloalkyl bedeuten, dadurch gekennzeichnet, dass man ein Anilinhydrohalogenid der Formel II

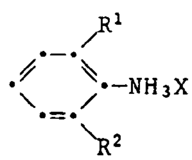

(II)

worin X für Chlor oder Brom steht und $R^1$ und $R^2$ die oben angegebene Bedeutung haben, in Gegenwart eines inerten, organischen Lösungsmittels mit Brom umsetzt.

2. Verfahren nach Anspruch 1, dadurch gekennzeichnet, dass $R^1$ und $R^2$ unabhhängig voneinander für $C_1$-$C_4$-Alkyl oder $C_5$-$C_6$-Cycloalkyl stehen.

3. Verfahren nach Anspruch 1 oder 2, dadurch gekennzeichnet, dass man als Lösungsmittel gesättigte Kohlenwasserstoffe oder chlorierte gesättigte Kohlenwasserstoffe verwendet.

4. Verfahren nach Anspruch 3, dadurch gekennzeichnet, dass man als Lösungsmittel Hexan, Cyclohexan, Methylcyclohexan, Dichlormethan, Trichlormethan, Tetrachlormethan oder 1,2-Dichlorethan verwendet.

5. Verfahren nach Anspruch 1, dadurch gekennzeichnet, dass sowohl $R^1$ als auch $R^2$ für Methyl, Ethyl oder i-Propyl oder $R^1$ für i-Propyl und $R^2$ für Cyclopentyl steht.

6. Verfahren nach einem der Ansprüche 1 bis 5, dadurch gekennzeichnet, dass man als Lösungsmittel 1,2-Dichlorethan oder Cyclohexan verwendet.

7. Verfahren nach einem der Ansprüche 1 bis 6, dadurch gekennzeichnet, dass man es bei einer Temperatur von -20°C bis +150°C durchführt.

8. Verfahren nach Anspruch 7, dadurch gekennzeichnet, dass man es bei einer Temperatur von -5 bis

+100°C durchführt.

9. Verfahren nach Anspruch 1, dadurch gekennzeichnet, dass man ein Anilinhydrohalogenid der Formel II in Gegenwart von 1,2-Dichlorethan oder Cyclohexan bei einer Temperatur von -5° bis +100°C mit Brom umsetzt.

## Claims

1. A process for the preparation of a 4-bromoaniline hydrobromide of formula I

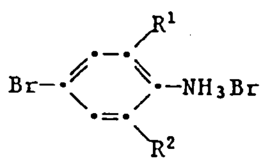

(I)

wherein $R^1$ and $R^2$ are each independently of the other $C_1$-$C_{10}$alkyl or $C_3$-$C_7$cycloalkyl, which process comprises brominating an aniline hydrohalide of formula II

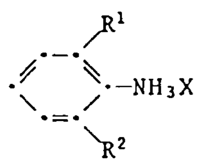

(II)

wherein X is chlorine or bromine and $R^1$ and $R^2$ are as defined above, in the presence of an inert organic solvent.

2. A process according to claim 1, wherein $R^1$ and $R^2$ are each independently of the other $C_1$-$C_4$alkyl or $C_5$-$C_6$cycloalkyl.

3. A process according to claim 1 or 2, wherein the solvent is a saturated hydrocarbon or a chlorinated saturated hydrocarbon.

4. A process according to claim 3, wherein the solvent is hexane, cyclohexane, methylcyclohexane, dichloromethane, trichloromethane, tetrachloromethane or 1,2-dichloroethane.

5. A process according to claim 1, wherein each of $R^1$ and $R^2$ is methyl, ethyl or isopropyl, or $R^1$ is isopropyl and $R^2$ is cyclopentyl.

6. A process according to any one of claims 1 to 5, wherein the solvent is 1,2-dichloroethane or cyclohexane.

7. A process according to any one of claims 1 to 6, which is carried out in the temperature range from -20°C to +150°C.

8. A process according to claim 7, which is carried out in the temperature range from -5° to +100°C.

9. A process according to claim 1, which comprises brominating an aniline hydrohalide of formula II in the presence of 1,2-dichloroethane or cyclohexane in the temperature range from -5° to +100°C.

## Revendications

1. Procédé de préparation des bromhydrates de 4-bromanilines de formule I

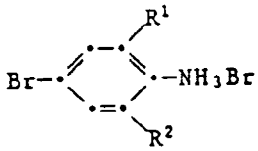

(I)

dans laquelle
$R^1$ et $R^2$ représentent chacun , indépendamment l'un de l'autre, un groupe alkyle en C 1-C 10 ou cycloalkyle en C 3-C 7, caractérisé en ce que l'on fait réagir un halogénhydrate d'aniline de formule II

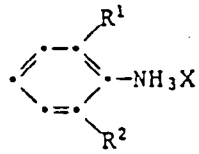

(II)

dans laquelle X représente le chlore ou le brome et $R^1$ et $R^2$ ont les significations indiquées ci-dessus, avec le brome en présence d'un solvant organique inerte.

2. Procédé selon la revendication 1, caractérisé en ce que $R^1$ et $R^2$ représentent chacun, indépendamment l'un de l'autre, un groupe alkyle en C 1-C 4 ou cycloalkyle en C 5-C 6.

3. Procédé selon la revendication 1 ou 2, caractérisé en ce que l'on utilise en tant que solvants des hydrocarbures saturés ou des hydrocarbures chlorés saturés.

4. Procédé selon la revendication 3, caractérisé en ce que l'on utilise en tant que solvant l'hexane, le cyclohexane, le méthylcyclohexane, le dichlorométhane, le trichlorométhane, le tétrachlorométhane ou le 1,2-dichloréthane.

5. Procédé selon la revendication 1, caractérisé en ce que $R^1$ et $R^2$ représentent tous deux des groupes méthyle, éthyle ou isopropyle ou bien $R^1$ représente un groupe isopropyle et $R^2$ un groupe cyclopentyle.

6. Procédé selon l'une des revendications 1 à 5 , caractérisé en ce que l'on utilise en tant que solvant le 1,2-dichloréthane ou le cyclohexane.

7. Procédé selon l'une des revendications 1 à 6, caractérisé en ce que l'on opère à une température de -20 à +150°C.

8. Procédé selon la revendication 7, caractérisé en ce que l'on opère à une température de -5 à +100°C.

9. Procédé selon la revendication 1, caractérisé en ce que l'on fait réagir un halogénhydrate d'aniline de formule II, en présence du 1,2-dichloréthane ou du cyclohexane, avec le brome à une température de -5 à +100°C.